# EUROPÄISCHE PATENTSCHRIFT

(11) **EP 3 291 757 B1**
(45) Veröffentlichungstag und Bekanntmachung des Hinweises auf die Patenterteilung: **25.09.2019**
(21) Anmeldenummer: 16726275.7
(22) Anmeldetag: 03.05.2016
(51) Int. Cl.: A61B 90/40, A61B 46/10, A61B 34/30

(54) **VORRICHTUNG ZUR POSITIONIERUNG VON STERILEN INSTRUMENTEN**
DEVICE FOR POSITIONING STERILE INSTRUMENTS
DISPOSITIF DE POSITIONNEMENT D'INSTRUMENTS STÉRILES

(30) Priorität: 03.05.2015 DE 202015003206 U
(43) Veröffentlichungstag der Anmeldung: 14.03.2018
(73) Patentinhaber: Isys Medizintechnik GmbH, 6370 Kitzbühel (AT)
(72) Erfinder: VOGELE, Michael, 86830 Schwabmünchen (DE)
(74) Vertreter: Fiener, Josef
(86) Internationale Anmeldenummer: PCT/EP2016/000722
(87) Internationale Veröffentlichungsnummer: WO 2016/177463

(56) Entgegenhaltungen:
- EP-A1- 0 516 582
- WO-A1-2014/162217
- DE-A1- 4 403 567
- DE-A1-102009 019 695
- DE-U1-202012 002 296
- US-A1- 2005 101 868
- US-A1- 2014 166 023

## Beschreibung

Die Erfindung betrifft eine Vorrichtung zur Positionierung von sterilen Instrumenten gemäss den oberbegrifflichen Merkmalen des Anspruchs 1, insbesondere zur gesteuerten Bewegung für einen roboterunterstützten operativen Eingriff in einer lokal um eine Eingriffstelle geschaffenen sterilen Umgebung.

Bei medizinischen Eingriffen ist die sterile Atmosphäre um die behandelte Körper- oder Hautstelle des Patienten von großer Bedeutung, um Infektionen zu vermeiden. Aus diesem Grund wird in Krankenhäusern und besonders in den Behandlungsräumen stets auf einen hohen Standard bezüglich der Sterilität geachtet, der sowohl für den Patienten, das behandelnde Personal und die verwendeten Instrumente bzw. Geräte gilt. Insbesondere bei minimal- oder mikro-invasiven Eingriffen, wie zum Beispiel bei einer perkutanen Nadelpunktion, ist die operativ behandelte Körperfläche sehr klein und teilweise sogar auf den jeweiligen Einstichpunkt beschränkt. Auch bei multiplen Nadel- oder Instrumentenplatzierungen ist die geforderte Sterilität vor allem in der lokal begrenzten Umgebung der entstandenen Hautöffnung notwendig.

Bei einer Vielzahl von chirurgischen Eingriffen ist die Benutzung eines ferngesteuerten Roboters vorteilhaft. So kommen Chirurgie-Roboter bei der Punktion oft zusammen mit Geräten zum Einsatz, die durch elektromagnetische Strahlung die genaue Einstichposition am Patienten und die richtige Einstichtiefe ermitteln, z. B. mit Röntgengeräten. Wegen der gesundheitsschädlichen Röntgenstrahlung kann die Behandlung oft nicht manuell durch einen Menschen ausgeführt werden, ohne dass die behandelnde Person länger der Strahlung ausgesetzt wird als es aus Gesundheitsgründen verantwortlich ist. Weiterhin erschwert die räumliche Einengung, die durch das Röntgengerät entsteht, einen manuellen Eingriff am Patienten. Darüber hinaus sind Roboter und Mikromanipulatoren bei der hochpräzisen Instrumentenpositionierung im 3D-Raum der menschlichen Hand deutlich überlegen. Aus diesen und anderen Gründen kommen Roboter und Telemanipulatoren vermehrt zum Einsatz z. B. um die durch das Röntgengerät bestimmte Eingriffs- und Zielposition am Patienten mit dem chirurgischen Eingriffsinstrument gezielt anzusteuern, aber auch um hochpräzise (mikrochirurgische) Eingriff am Patienten erst zu ermöglichen. Chirurgische Roboter, die eine Einführung und Manipulation von Nadeln und nadelartigen (Mikro-) Instrumenten sowie von therapeutischen und diagnostischen (Mikro-)Apparaten erlauben, sind für die Anwendung mit bildgebenden Verfahren (wie CT, C-Bogen, Röntgen, MRT, Ultraschall etc.) durch die relativ geringe Größe der Roboter und der örtlichen Beschränktheit der zu behandelten Stelle somit besonders geeignet.

Um eine exakte Platzierung des Eingriffsinstruments in der Bildgebung mit anschließender Bildverarbeitung der bestimmten Stelle zu ermöglichen, sollte der Abstand der Halterungen des Roboters zu der Nadel möglichst gering gehalten werden, was jedoch die Gefahr einer Verunreinigung der entstehenden Wunde durch den Roboter birgt.

Derartige Roboter, die ferngesteuert genaue Punktionen an einem Patienten vornehmen können, sind beispielsweise in der EP 1 722 698 B1 oder US-A-5 176 689 beschrieben. Um die Nadel und die Einstichstelle vor von dem Roboter herrührenden, möglichen Verunreinigungen zu schützen, wurden bereits verschiedene Möglichkeiten vorgeschlagen, um den Roboter oder Teile davon abzudecken. Die Nadel bewegt sich dann, von einem Schutz vom Roboter getrennt, im Raum mit etwa der Sterilität, die im Behandlungsraum herrscht. Solche Beispiele von am Roboter angebrachten Schutzvorrichtungen sind in der US 2006/0235436 A1 oder WO 2014/127984 A1 gezeigt. Diese Ausführungen haben den Nachteil, dass zumindest Teile des Roboters luftdicht abgedeckt werden müssen und dieser Schutz nach einer gewissen Zeit gewechselt werden muss, was eine zeitintensive Prozedur darstellt.

Aus der WO 2010/121107 A1 ist ein chirurgischer Manipulator bekannt, der als Gesamtheit steril verpackt (ähnlich auch in der US-A-6 132 368) und von außerhalb der sterilen Verpackung drahtlos gesteuert werden kann. An der Außenseite der sterilen Verpackung des Manipulators wird das chirurgische Werkzeug angebracht. Diese Anordnung lässt ein hohes Maß an Sterilität des Manipulators zu, hat jedoch die Nachteile, dass zur Manipulation des instruments elektromagnetische Strahlen benutzt werden und das chirurgische Werkzeug außerhalb der sterilen Verpackung um den Manipulator herum angebracht wird.

Die WO 2014/1622217 beschreibt eine Vorrichtung zur Positionierung von Instrumenten in einer sterilen Hülle. Dabei sind Durchführungen für elektrische Verbinder vorgesehen. Zur weiteren Fixierung gegenüber dem Patienten sind keine Angaben ersichtlich. Dies gilt auch für die US 2005/101868, welche eine Vorrichtung zur Führung von Instrumenten zeigt, wobei ein Handgriff vorgesehen ist. Die DE 44 03 567 A1 beschreibt zudem eine Vorrichtung mit einer sterilen Hülle zur Durchführung endoskopischer Operationen.

Demzufolge liegt der Erfindung die Aufgabe zugrunde, eine einfache Vorrichtung, insbesondere für roboterunterstützte Eingriffe zu schaffen, die in einer lokalen Umgebung höchster Sterilität ermöglicht und nicht auf die Anwendung eines bestimmten Robotertyps begrenzt ist.

Die Grundidee der Erfindung liegt darin, das operative Werkzeug steril durch eine Hülle von der Umgebung (inkl. Roboter) lokal zu schützen und die Vorrichtung derart zu gestalten, dass während des operativen Eingriffs die sterile Atmosphäre in einem begrenzten Volumen um die Eingriffsstelle herum erhalten bleibt. Es beschreibt damit eine Anordnung, die lokal an der Stelle des Eingriffs eine sterile Atmosphäre ermöglicht, die nicht durch den chirurgischen Eingriff und durch die Reinheitsbedingungen außerhalb der Anordnung beeinflusst wird.

Die vorgeschlagene Vorrichtung dient der Positionierung, insbesondere der Einführung und Manipulation von Nadeln und (Mikro-) Instrumenten sowie dem Positionieren von therapeutischen und diagnostischen (Mikro-)Apparaten wie z. B. Punktions-, Injektions-, Ablationsnadeln oder Endoskopen oder ähnlichem zu einem Patienten hin. Hierbei ist die "ballonartige" Hülle zumindest auf ihrer Innenseite steril und umhüllt ein steriles Instrument wenigstens teilweise, vorzugsweise komplett oder größtenteils. Zudem ist an der Hülle mindestens eine gasdichte Durchführung vorgesehen, welche z. B. zur Ankoppelung einer Instrumentenhalterung dienen kann. Hierbei ist eine solche Instrumentenhalterung ebenso wie das Instrument selbst steril in der Hülle aufgenommen und durch Bewegung der Instrumenthalterung außerhalb der Hülle in mindestens zwei Raumrichtungen gesteuert bewegbar. Die Instrumenthalterung besitzt vorzugsweise außerhalb der Hülle Ankoppelteile (Schnellverschlüsse) zu einen externen Manipulator hin, der gemäß den o.g. Stand der Technik ausgeführt sein kann, insbesondere gemäß der EP 1 722 698 B1

Die Komponenten der Vorrichtung können gemeinsam oder separat durch bekannte Sterilisationsverfahren sterilisiert werden und können in einer sterilen Verpackung und Transportbox sicher steril gelagert werden. Der Zusammenbau der einzelnen Komponenten kann in unsteriler Umgebung vor dem Sterilisationsvorgang oder direkt vor dem Eingriff in steriler Umgebung erfolgen.

Bevorzugt weist das sterile Instrument mindestens eine nadelförmige Spitze aufweist, mit der eine zum Patienten hin angeordnete Membran perforierbar ist. Um eine unabsichtliche Perforation der sterilen Hülle zu verhindern kann die Instrumentenspitze mit einer Schutzkappe versehen sein, welche vor dem Eingriff durch die Hülle hindurch abgelöst werden kann. Die Anbringung der Hülle am Patienten, genauer an der Eingriffsstelle erfolgt bevorzugt mit einer Klebefläche. Die Eingriffsstelle wird vor dem Eingriff lokal gereinigt, zur zusätzlichen Erhöhung der Sterilität oder um sich den Reinigungsprozess an der Eingriffsstelle zu sparen, kann die Klebefläche mit antiviraler, antibakterieller Beschichtung versehen sein. Um ein leichteres Handling der sterilen Hülle und insbesondere die Anbringung der Klebefläche am Patienten zu ermöglichen, kann die Klebefläche durch mechanische Strukturen verstärkt sein und griffartige Ausstülpungen aufweisen. Durch diese platzsparenden Verstärkungen und Angriffsmöglichkeiten wird eine sauberere/plane und sterile Anbringung der Klebefläche auf dem Patienten auch an schwer zugänglichen Körperregionen und bei engen Platzverhältnissen ermöglicht. Das durch die Hülle steril gehaltene Instrument kann auch über einen Finger- oder Handgriff gesteuert werden, insbesondere bei kurzen Eingriffen ohne längere Strahlenbelastung oder beim Einsatz mit bildgebenden Verfahren, die keine Strahlenbelastung mit sich bringen (wie z.B. Ultraschall, MRT). Der Handgriff, insbesondere an dem der Spitze gegenüberliegenden Ende kann hierzu außerhalb der Hülle angeordnet sein, wobei der Hauptteil des Instruments durch eine (weitere) Durchführung in die Hülle hineinragt. Diese Bauart bietet sich z. B. an, wenn das sterile Instrument am vorderen Ende verschlossen ist oder eine Hohlnadel ist, die außerhalb der Hülle einen sterilen Anschluss für die Zufuhr von Wirkstoffen in die Hohlnadel aufweist.

Als besondere Art der Durchführung ist bei der Vorrichtung eine Membran vorgesehen, die das Instrument an der Durchstichstelle gasdicht umschließt. Bevorzugt ist dabei die Membran von mindestens einer an der Außenseite der Membran bzw. Hülle angebrachten, abziehbaren Schutzfolie abgedeckt, wobei die Membran und/oder Schutzfolie wie oben beschrieben von einem Haltering stabilisiert sein kann. Zur Detektion und Referenzierung des Halterings bzw. der Schutzhülle und des Instruments im Raum in Relation zum Patienten bzw. der Umgebung können aktive und passive Marker an sämtlichen Komponenten der Vorrichtung angebracht sein. Zudem beinhaltet die Hülle bevorzugt ein Einlassventil, das zur Gaszufuhr eines sterilisierenden Gases und/oder eines Inertgases in die Hülle dient, vorzugsweise zur Erzeugung eines Überdrucks innerhalb der Hülle, welche zur freien Beweglichkeit der "aufgeblasenen" Hülle eine Entlüftungsöffnung besitzt. Das Einlassventil oder eine gleichwirkende semipermeable Membran kann beim Platzieren des Instruments gleichzeitig als Überdruckventil oder Auslassventil dienen.

Von besonderer, eigenständiger Bedeutung ist die erfindungsgemäße Ausführung der Hülle mit einer ringförmigen Klebefläche, mit der die Hülle zusammen mit dem (sterilen) Instrument an der Eingriffstelle gasdicht fixiert werden kann. Hierzu muss (neben der üblichen Desinfektion) lediglich die Schutzfolie abgezogen werden, so dass eine rasche Bereitstellung ermöglicht wird. Hierzu trägt auch bei, dass die Hülle mit Klebefläche und wenigstens einer Schutzfolie zusammen mit dem Instrument in einem Transportbehälter steril verpackt sind (siehe oben).

Nachfolgend wird ein Ausführungsbeispiel der Vorrichtung anhand der Zeichnung näher erläutert. Hierbei zeigen:
Fig. 1 eine Übersichtdarstellung der Vorrichtung;
Fig. 2 eine vergrößerte Perspektivansicht analog zu Fig. 1;
Fig. 3 eine weitere Vergrößerung gemäß Fig. 1 bzw. 2; und
Fig. 4 einen Transportbehälter hierzu.

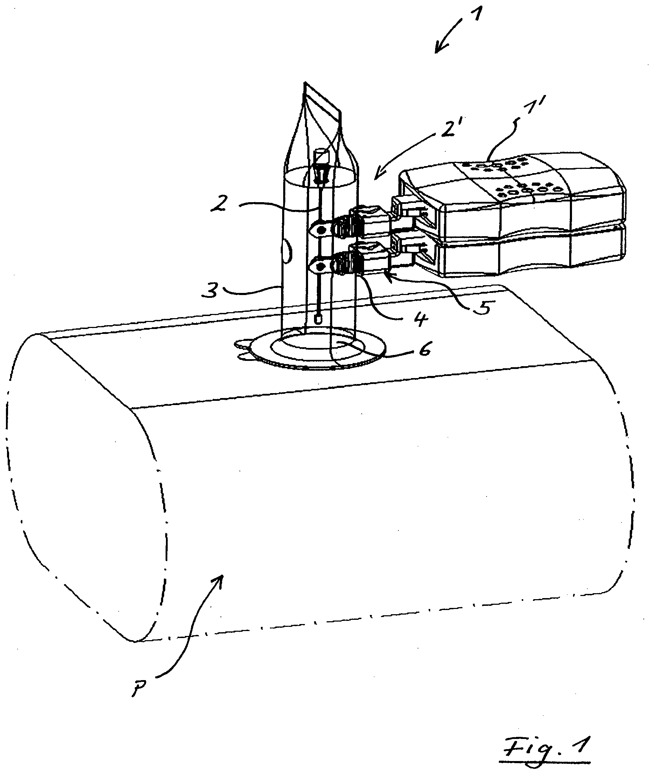

In Fig. 1 ist eine vorstehend beschriebene Vorrichtung 1 auf einem Körperteil eines Patienten aufgesetzt, z. B. einer Wirbelsäule oder einen Kniegelenk. Das bereits sterile Instrument 2, beispielsweise eine Punktionsnadel oder Injektionsnadel, ist durch eine ebenfalls sterile Halterung 2' in einer zumindest an der Innenseite sterilen Hülle 3 angebracht. Die Hülle 3 ist in Art eines Ballons bevorzugt transparent ausgebildet. Gasdichte Durchführungen 4 in der Hülle 3 führen die sterile Instrumentenhalterung 2' zu Ankoppelteilen 5, an denen ein chirurgischer Roboter bzw. Manipulator 1' durch geeignete Anschlüsse befestigt werden kann. Somit kann das operative Werkzeug (Instrument 2) innerhalb des sterilen Innenraums vom Außenraum der Hülle 3 aus bewegt werden.

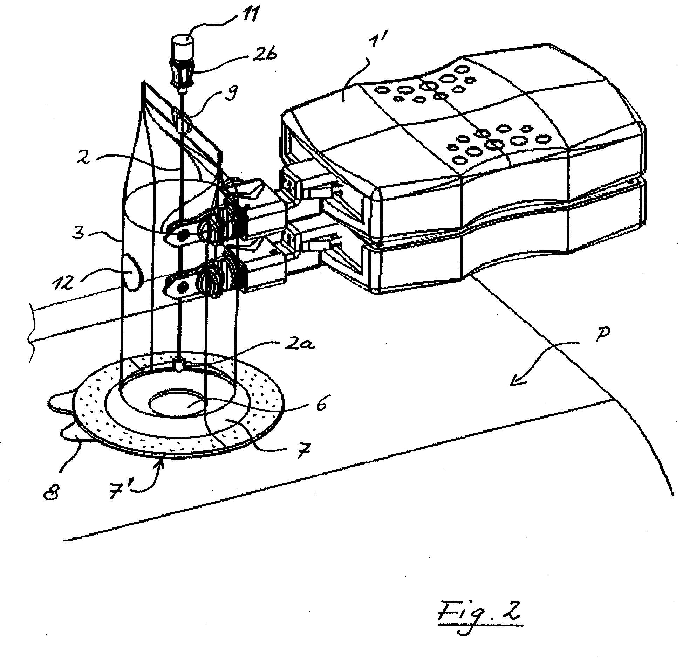

Wie in Fig. 2 dargestellt, kann an der Spitze 2a des Instruments 2 als operatives Werkzeug, hier am unteren Ende der Hülle 3 eine Membran 6 vorgesehen sein, die von der Spitze 2a (nach Durchdringen oder Entfernen eines Spitzenschutzes) durchstochen werden kann. Die Membran 6 ist ebenso wie die Durchführung 4 gasdicht an der Hülle 3 angebracht. An der Hülle 3 ist um die Membran 6 herum weiterhin ein Verstärkungs- oder Haltering 7 zur Stabilisierung angebracht, der mit einer hier punktiert dargestellten Klebefläche 7' an der vorher desinfizierten Punktionsstelle des Patienten P befestigt wird. Mindestens eine abziehbare Schutzfolie 8 schützt die Membran 6 dabei vor etwaigen Verunreinigungen vor dem Einsatz.

Gegenüber der Membran 6 ist am oberen Ende der Hülle 3 eine gasdichte Manschette 9 als Durchführung angebracht, durch die das Instrument 2 in den Außenraum der Hülle 3 ragen kann. Dabei kann an der Nadel ein Hand- bzw. Fingergriff 2b vorgesehen sein, um die Nadel ggf. manuell und feinfühlig zu bewegen. Ein Anschluss 11 am oberen Ende des sich im Außenraum der Hülle 3 befindlichen Instruments 2 ermöglicht eine Medikamentenzufuhr durch eine Hohlnadel zum Patienten. Der herausstehende Teil der Nadel kann wahlweise auch mit einem Kuppelteil versehen sein, um mit diesem eine robotergesteuerte Bewegung an der Nadel vorzunehmen, z. B. eine axiale Vorschubbewegung.

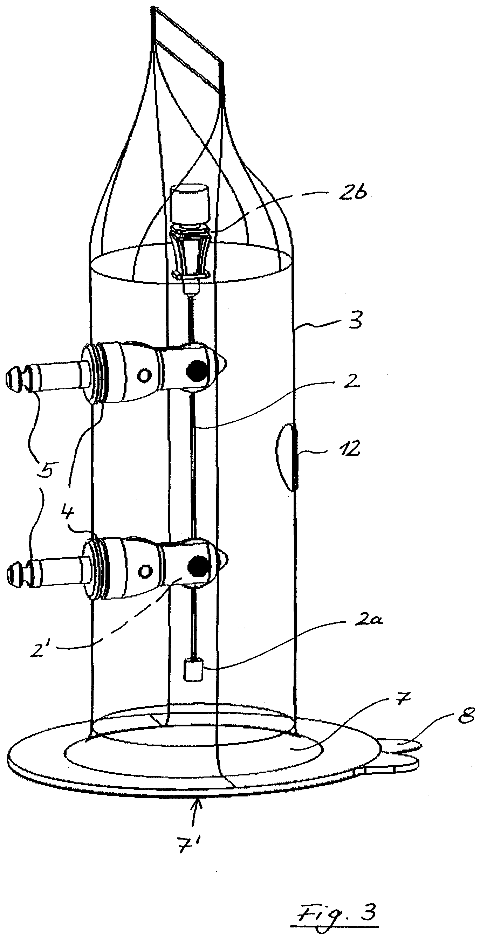

Solche Ankuppelteile 5 in Form von Klinkenstecker sind vergrößert in Fig. 3 gezeigt, Um die hohe Sterilität innerhalb der Hülle 3 zu unterstützen kann, kann über ein (optional mit einem Filter versehenen) Einlassventil 12 durch Zufuhr eines geeigneten Gases in der Hülle 3 ein leichter Überdruck erzeugt werden, der das Eindringen von keimbeladener Außenluft während der Behandlung verhindert. Als geeignetes Gas kann beispielweise gereinigte Luft oder reines Stickstoff verwendet werden. An der Hülle 3 kann eine weitere Öffnung 13 vorgesehen sein, über welche das zugeführte Gas aus der Hülle 3 entweichen, wenn beispielsweise der Gasdruck in der Hülle diese so stark stabilisiert, dass eine Bewegung der Nadel 2 relativ zur Hülle 3 und damit hin zur Membran 6 nur noch erschwert möglich ist. Eine gewünschte Desinfizierung vor und ggf. nach dem Gebrauch der Vorrichtung 1 kann durch Zufuhr eines desinfizierenden Gases durch das Ventil 12 ermöglicht werden. Anstelle eines Ventils können aber auch semipermeable Hüllmaterialien verwendet werden, die ein Ein- oder Ausströmen von Gasen erlauben. Alternativ zur Gassterilisation können auch gasfreie Sterilisationsverfahren (z. B. Bestrahlung) zum Einsatz kommen.

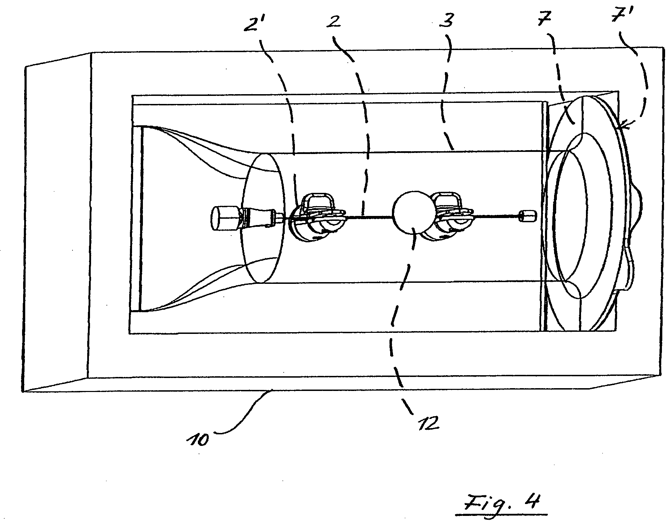

Wie in Fig. 4 dargestellt kann über das Ventil 12 auch die gesamte Vorrichtung 1 (mit Hülle 3, Instrument 2 etc) sterilisiert werden und dann in einen Transportbehälter 10 verpackt werden. Damit sind die Bauteile zur späteren Eingriffstelle am Patienten sicher steril gehalten. Daher erlaubt es die Vorrichtung, auch roboterunterstützte Eingriffe an einem Patienten in einem sterilen Raum vorzunehmen, der lokal um die Hautöffnung des Patienten geschaffen ist. Der Behandlungsroboter, die übrigen Instrumente und Personen, die sich im Behandlungsraum befinden, kommen in keinen Luftkontakt mit der zu behandelten Stelle am Patienten und mit der desinfizierten Nadel. Damit ist erreicht, dass etwaige Probleme der Sterilität im Behandlungsraum wenig oder keinen Einfluss auf die Keimfreiheit am lokal begrenzten Behandlungspunkt haben.

Neben dem Ziel der absoluten Keimfreiheit (Infektionen z. B. durch gefährliche Hospitalkeime wie MRSA werden zu einem zunehmenden Problem in Krankenhäusern und die Hygienevorschriften werden immer strikter) soll diese Vorrichtung die Eingriffszeit maximal verkürzen und die Kosten für sterile Abdeckungen/Bekleidungen/Einwegartikel etc. maximal reduzieren.

Darüber hinaus ermöglicht diese Vorrichtung die Durchführung steriler (mikro)invasiver Eingriffe, selbst in unsteriler Umgebung. Besonders in Ländern, in denen aus Geldgründen keine sterilen OP-Räume und OP-Bedingungen vorhanden sind, werden somit eine Vielzahl von Eingriffen unter höchsten Sterilitätsstandards möglich. Aber auch in reicheren Ländern sind die hohen Hygienestandards in vielen medizinischen Bereichen kaum mehr finanzierbar und erfüllbar. Insbesondere bei bildgestützten Operationen erfüllen viele Räumlichkeiten nicht die erforderlichen Hygienestandards. Dies hat zur Folge, dass viele Räumlichkeiten entweder aufwendig und kostenintensiv saniert werden müssen oder für (bildgestützte) (Roboter-)Interventionen nicht mehr genutzt werden dürfen. All diese Probleme werden durch die vorgeschlagene Vorrichtung effizient und kostengünstig gelöst.

## Patentansprüche

1. Vorrichtung zur Positionierung von sterilen Instrumenten, insbesondere von Punktionsnadeln, Injektionsnadeln oder Operationssonden, zu einem Patienten hin, mit einer zumindest auf ihrer Innenseite sterilen Hülle (3) und dem sterilen Instrument (2), wobei die Hülle (3) das sterile Instrument (2) wenigstens teilweise umhüllt, und mit mindestens einer an der Hülle (3) angebrachten, gasdichten Durchführung (4), wobei das sterile Instrument (2) mindestens eine sterile Instrumentenhalterung (2') aufweist, die durch die Durchführung (4) hindurch zu Ankoppelteilen (5) geführt ist und durch Bewegung der Instrumentenhalterung (2') von außerhalb der Hülle (3) in mindestens zwei Raumrichtungen gesteuert bewegbar ist,
**dadurch gekennzeichnet, dass**
die Hülle (3) zum Patienten (P) hin eine ringförmige Klebefläche (7') aufweist, mit der die Hülle (3) zusammen mit dem Instrument (2) an einer Eingriffsstelle gasdicht fixierbar ist.

2. Vorrichtung nach Anspruch 1, **dadurch gekennzeichnet, dass**
das sterile Instrument (2) mindestens eine nadelförmige Spitze (2a) aufweist, mit der eine zum Patienten (P) hin angeordnete, zur Hülle (3) gehörigen Membran (6) perforierbar ist, insbesondere nach Abnehmen eines Spitzenschutzes.

3. Vorrichtung nach Anspruch 1 oder 2, **dadurch gekennzeichnet, dass**
die Instrumentenhalterung (2') außerhalb der Hülle (3) Ankoppelteile (5) zu einen externen Manipulator (1') hin aufweist.

4. Vorrichtung nach einem der Ansprüche 1 bis 3, **dadurch gekennzeichnet, dass**
das sterile Instrument (2) außerhalb der Hülle (3) einen Handgriff (2b) aufweist und/oder direkt an einen externen Manipulator (1') anschließbar ist.

5. Vorrichtung nach einem der Ansprüche 1 bis 4, **dadurch gekennzeichnet, dass**
das sterile Instrument (2) eine Hohlnadel ist, die außerhalb der Hülle (3) einen Anschluss (11) für die Zufuhr von Wirkstoffen in die Hohlnadel aufweist.

6. Vorrichtung nach Anspruch 2 und einem der Ansprüche 3 bis 5, **dadurch gekennzeichnet, dass** die Membran (6) das Instrument (2) an einer Durchstichstelle gasdicht umschließt.

7. Vorrichtung nach Anspruch 2 und einem der Ansprüche 3 bis 6, **dadurch gekennzeichnet, dass** die Membran (6) von mindestens einer an der Außenseite der Membran (6) angebrachten, abziehbaren Schutzfolie (8) abgedeckt ist, wobei die Membran (6) und/oder Schutzfolie (8) von einem Haltering (7) stabilisiert ist.

8. Vorrichtung nach einem der Ansprüche 1 bis 7, **dadurch gekennzeichnet, dass**
die Hülle (3) ein Einlassventil oder eine semipermeable Membran (12) zur Gaszufuhr eines sterilisierenden Gases und/oder eines Inertgases in die Hülle (3) enthält, vorzugsweise zur Erzeugung eines Überdrucks innerhalb der Hülle (3) sowie vorzugsweise eine Entlüftungsvorrichtung aufweist.

9. Vorrichtung nach einem der Ansprüche 1 bis 8, **dadurch gekennzeichnet, dass** die Hülle (3) mit Klebefläche (7') und wenigstens einer Schutzfolie (8) zusammen mit dem Instrument (2) in einem Transportbehälter (10) steril verpackt sind.

10. Vorrichtung nach einem der Ansprüche 1 bis 9, **dadurch gekennzeichnet, dass** mindestens an einer der Komponenten der Vorrichtung (1) ein Marker angebracht ist.

## Claims

1. Device for positioning sterile instruments, in particular puncture needles, injection needles or surgical probes, with respect to a patient, with a sheath (3) which is sterile at least on its inner side and an instrument (2), wherein the sheath (3) at least partially encloses the sterile instrument (2), and with at least one gas-tight passage (4) mounted at the sheath (3), wherein the sterile instrument (2) has at least one sterile instrument holder (2') which passes through the passage (4) to coupling parts (5) and is movable in a controlled manner in at least two spatial directions by movement of the instrument holder (2') outside the sheath (3),
**characterized in that**
the sheath (3) has an annular adhesive surface (7'), directed towards the patient (P), for fixing the sheath (3) together with the instrument (2) on an interventional site in a gas-tight manner.

2. Device according to claim 1, **characterized in that** the sterile instrument (2) has at least one needle-shaped tip (2a) with which a membrane (6) belonging to the sheath (3) and arranged towards the patient (P) can be perforated, in particular after removal of a tip protector.

3. Device according to claim 1 or 2, **characterized in that** the instrument holder (2') has coupling parts (5) to an external manipulator (1') outside the sheath (3).

4. Device according to one of claims 1 to 3, **characterized in that** the sterile instrument (2) has a handle (2b) and/or can be directly connected to an external manipulator (1') outside the sheath (3).

5. Device according to one of claims 1 to 4, **characterized in that** the sterile instrument (2) is a hollow needle, which has a connector (11) outside the sheath (3) for supplying substances into the hollow needle.

6. Device according to claim 2 and one of claims 3 to 5, **characterized in that** the membrane (6) surrounds the instrument (2) at a puncture site in a gas-tight manner.

7. Device according to claim 2 and one of claims 3 to 6, **characterized in that** the membrane (6) is covered by at least one peelable protective film (8) on the outside of the membrane (6), wherein the membrane (6) and/or the protective film (8) is stabilized by a retaining ring (7).

8. Device according to one of claims 1 to 7, **characterized in that** the sheath (3) includes an inlet valve or a semi-permeable membrane (12) for supplying sterilizing gas and/or inert gas into the sheath (3), preferably for generating an overpressure within the sheath (3), and preferably including a venting device.

9. Device according to one of claims 1 to 8, **characterized in that** the sheath (3) having the adhesive surface (7') and at least one protective film (8) is packaged together with the instrument (2) in a transport container (10) in a sterile manner.

10. Device according to one of claims 1 to 9, **characterized in that** a marker is attached to at least one of the components of the device (1).

## Revendications

1. Ensemble pour positionner par rapport à un patient des instruments stériles, notamment des aiguilles de ponction, des aiguilles d'injection ou des sondes opératoires, l'ensemble présentant au moins sur son côté intérieur une enveloppe stérile (3) et l'instrument stérile (2), l'enveloppe (3) englobant au moins partiellement l'instrument stérile (2), et présentant au moins un passage (4) étanche aux gaz ménagé sur l'enveloppe (3),
l'instrument stérile (2) présentant au moins un support (2') d'instrument stérile relié à des pièces d'accouplement à travers le passage (4) et apte à être déplacé de manière contrôlée dans au moins deux directions de l'espace depuis l'extérieur de l'enveloppe (3) par déplacement du support d'instrument,
**caractérisé en ce que**
du côté du patient (P), l'enveloppe (3) présente une surface adhésive (7') de forme annulaire par laquelle l'enveloppe (3) et l'instrument stérile (2) peuvent être fixés de manière étanche aux gaz à l'emplacement d'une intervention.

2. Ensemble selon la revendication 1, **caractérisé en ce que** l'instrument stérile (2) présente au moins une pointe (2a) en forme d'aiguille par la quelle une membrane faisant partie de l'enveloppe (3) et disposée vers le patient (P), en particulier après avoir enlevé un protège-pointe.

3. Ensemble selon les revendications 1 ou 2, **caractérisé en ce que** le support (2') d'instrument stérile présente à l'extérieur de l'enveloppe (3) des pièces d'accouplement (5) à un manipulateur externe (1').

4. Ensemble selon l'une des revendications 1 à 3, **caractérisé en ce que** l'instrument stérile (2) présente à l'extérieur de l'enveloppe (3) une poignée (2b) et/ou peut être raccordé directement à un manipulateur externe (1').

5. Ensemble selon l'une des revendications 1 à 4, **caractérisé en ce que** l'instrument stérile (2) est une aiguille creuse qui présente à l'extérieur de l'enveloppe (3) un raccord (11) permettant l'apport de substances actives dans l'aiguille creuse.

6. Ensemble selon la revendication 2 et l'une des revendications 3 à 5, **caractérisé en ce que** la membrane (6) entoure l'instrument stérile (2) de manière étanche aux gaz en un emplacement de perforation.

7. Ensemble selon la revendication 2 et l'une des revendications 3 à 5, **caractérisé en ce que** la membrane (6) est recouverte par au moins une feuille de protection (8) étirable placée sur la face extérieure de la membrane (6), la membrane (6) et/ou la feuille de protection (8) étant stabilisées par une bague de maintien (7).

8. Ensemble selon l'une des revendications 1 à 7, **caractérisé en ce que** l'enveloppe (3) contient une soupape d'admission ou une membrane semi-perméable (12) pour l'amenée d'un gaz de stérilisation et/ou d'un gaz inerte dans l'enveloppe (3) et de préférence pour former une surpression à l'intérieur de l'enveloppe (3), ainsi qu'un ensemble d'évacuation des gaz.

9. Ensemble selon l'une des revendications 1 à 7, **caractérisé en ce que** l'enveloppe (3), la surface adhésive (7') et au moins une feuille de protection (8) sont emballées de manière stérile avec l'instrument stérile (2) dans un récipient de transport (10).

10. Ensemble selon l'une des revendications 1 à 7, **caractérisé en ce que** un repère est placé sur au moins l'un des composants de l'ensemble (1).
